# EUROPEAN PATENT APPLICATION

(11) **EP 4 206 186 A1**
(43) Date of publication of application: **05.07.2023**
(21) Application number: 20951170.8
(22) Date of filing: 16.11.2020
(51) Int. Cl.: C07C 265/14, C07C 263/04, C07C 271/20, C07C 269/04

(54) **PREPARATION METHOD FOR 1,5-PENTANE DIISOCYANATE**

(30) Priority: 27.08.2020 CN 202010879607
(71) Applicant: INSTITUTE OF PROCESS ENGINEERING, CHINESE ACADEMY OF SCIENCES, Haidian District Beijing 100190 (CN)
(72) Inventor: WANG, Liguo, Beijing 100190 (CN); XU, Shuang, Beijing 100190 (CN); LI, Huiquan, Beijing 100190 (CN); HE, Peng, Beijing 100190 (CN); CAO, Yan, Beijing 100190 (CN); CHEN, Jiaqiang, Beijing 100190 (CN); ZHENG, Zheng, Beijing 100190 (CN); WANG, Xue, Beijing 100190 (CN); ZHAO, Xuefeng, Beijing 100190 (CN)
(74) Representative: Cabinet Beaumont
(86) International application number: PCT/CN2020/129047
(87) International publication number: WO 2022/041502

(57) **Abstract**

Disclosed herein is a method for preparing 1,5-pentane diisocyanate, comprising the following steps: (1) mixing 1,5-pentanediamine, a carbonylation agent, a first solvent and a catalyst for a carbonylation reaction, performing solid-liquid separation, and obtaining a 1,5-pentamethylene dicarbamate reaction solution; and (2) purifying the 1,5-pentamethylene dicarbamate reaction solution, then mixing same with a second solvent for a pyrolysis reaction, and obtaining 1,5-pentane diisocyanate.

## Description

### TECHNICAL FIELD

The present application belongs to the technical field of isocyanate synthesis, and relates to a method for preparing aliphatic diisocyanate, and for example, relates to a method for preparing 1,5-pentane diisocyanate.

### BACKGROUND

Polyurethane materials prepared by aliphatic and alicyclic diisocyanate (ADI) have excellent mechanical property, outstanding chemical stability and excellent weathering resistance, which have been widely used in high-end building exterior wall coatings, automotive coatings, product shell coatings, industrial equipment pipes, thermal insulation materials, foamed plastics, synthetic fibers, coatings and solid elastic materials, as well as light industrial products that are closely related to human life. Compared with the aromatic isocyanate, such as MDI and TDI, most species of in the ADI series have better performance and lower toxicity, and are mainly used in the production of high-end or special polyurethane products.

1,5-Pentane diisocyanate (PDI) belongs to the ADI series, which has high reactivity and can be cured at a low temperature in a short time. The PDI improves energy efficiency, and can also improve the materials in chemical resistance, wear resistance and luster. PDI can raise nylon, isocyanate and other fields to a higher level.

1,5-Pentanediamine (PDA), the key raw material in the PDI production, can be prepared by biological method. A series of research achievements has been achieved in biological fermentation PDA preparation, and the committed decarboxylation step requires the use of L-lysine decarboxylase. By strengthening the PDA metabolic pathway and developing new host strains, the construction of engineering strains is realized which can efficiently synthesize PDA, completely solving the industrial bottleneck of bio-based PDA, and basically realizing the industrial production of bio-based 1,5-pentanediamine raw materials. 1,6-Hexanediamine (HDA) is a key raw material in the synthesis of 1,6-hexamethylene diisocyanate (HDI). HDA is synthesized from petrochemical raw materials. Compared with HDA, the PDA biosynthesis has the advantages of low cost, renewable raw materials and environmental protection, which avoids the defects of complex HDA synthesis process, high technical difficulty and non-renewable raw materials.

With the large-scale production of biosynthesis PDA raw materials, the synthesis research and industrialization development of PDI will also attract extensive attention. Like production processes of other isocyanate, the synthesis of PDI is divided into phosgene method and non-phosgene method. The phosgene method is a commonly used production method in industry, but the phosgene used in the process of preparing isocyanate is a highly toxic raw material, and a large amount of hydrogen chloride will be released during the reaction, which can easily cause equipment corrosion and environmental pollution. From the perspective of environmental protection, it is necessary to develop the non-phosgene technology.

The preparation of PDI from PDA is known, which can be achieved by a non-phosgene method (T. Lesiak, K. Seyda, Journal für Praktische Chemie (Leipzig), 1979, 321(1), 161-163) or by phosgene reaction (e.g. W. Siefken, Justus Liebigs Ann. Chem. 562, 1949, Page 25 and the subsequent pages, or DE2625075A1).

In the case of the non-phosgene preparation cited above, PDA first reacts with formic acid to produce formamide, and then oxidizes with halogens in the presence of tertiary amine to produce PDI. The disadvantage of this method is that it is a complex two-stage method, and forms lots of by-products, reduces the yield and increases the purification complexity, thus reducing the economic feasibility of this method. DE2625075A1 provides a method for preparing carbamoyl chloride and isocyanate. In this method, the salt of primary amine in solid form reacts with phosgene in the presence of a liquid. The disadvantage of this method is that it is also a multi-stage method. In the first stage, the amine salt is first prepared in a solvent, and then this solvent must be removed before the amine salt reacts with phosgene, for example, by filtration or centrifugation, and then drying is performed. This method is time-consuming and expensive, which reduces the economic feasibility of this method.

DE1900514A1 provides a two-stage preparation method for PDI, in which caprolactam is converted to hydroxamic acid and then subjected to phosgenation to prepare PDI, but the yield of PDI prepared from caprolactam is only about 32%.

WO2008015134A1 provides a method for preparing PDI, in which bio-based lysine is converted into PDA, and then converted into PDI. PDA can be converted into PDI in a non-phosgene manner or in the presence of phosgene, and the latter variant can be in the liquid phase or in the gas phase. The document fails to mention the possible interfering impurities in PDI or the methods of avoiding or minimizing them.

EP2015071438 provides a method for preparing PDI by reacting PDA with phosgene in a gas phase. The crude PDI material with very low contents of 5-chloropentyl isocyanate (CPI), N-carbamoylpiperidin (C6-Im) and N-carbamoyltetrahydropyridine (C6-Az) having two isomerides can be obtained by a reaction in the gas phase above the boiling point temperature of the PDA.

The non-phosgene method of PDI is mainly the carbamate cleavage method, which is still in the laboratory research stage. CN108689884A reports a method for preparing 1,5-pentane diisocyanate. In this method, the extraction mixture in the PDA production process is directly subjected to carbamate reaction, and distilled to remove the extraction solvent and then subjected to thermal cleavage to obtain 1,5-pentane diisocyanate. The process is complicated, and the PDI yield is about 50% which needs to be improved. Its industrial application has limitations and it is difficult to realize industrial production.

There are some defects in the related technology, such as high toxicity of raw materials, high cost of raw materials and complicated process. Therefore, it is of great significance to develop a non-phosgene preparation method which is green and safe and has simple process and high PDI yield.

### SUMMARY

An object of the present application includes providing a method for preparing 1,5-pentane diisocyanate. The preparation method has mild reaction condition, simple operation, no pollution, and little potential safety hazard, with high yield and purity of intermediate PDC and final product PDI.

Therefore, the present application provides a method for preparing 1,5-pentane diisocyanate, which includes the following steps:
(1) mixing 1,5-pentanediamine, a carbonylation agent, a first solvent and a catalyst, performing a carbonylation reaction, and performing solid-liquid separation to obtain a pentane-1,5-dicarbamate reaction solution; and
(2) subjecting the pentane-1,5-dicarbamate reaction solution obtained in step (1) to a purification treatment, mixing pentane-1,5-dicarbamate after the purification treatment with a second solvent, and performing a thermal decomposition to obtain the 1,5-pentane diisocyanate.

In the present application, compared with the phosgene route, the synthetic route of the preparation method avoids the use of highly toxic phosgene, and is a safe, non-toxic and harmless green production process.

As an optional technical solution in the present application, the carbonylation agent in step (1) has a structure as shown in Formula I: 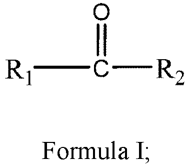 wherein Ri includes any one of amino, methoxy, ethoxy, n-propoxy, isopropoxy, n-butoxy or isobutoxy, and R₂ includes any one of amino, methoxy, ethoxy, n-propoxy, isopropoxy, n-butoxy, isobutoxy, sec-butoxy or tert-butoxy.

In the present application, the carbonylation agent can be any one of the compounds represented by Formula I, or a combination of at least two of the compounds represented by Formula I.

Optionally, a molar ratio of the carbonylation agent in step (1) to the 1,5-pentanediamine is (2-20):1, such as 3:1, 4:1, 5:1, 6:1, 7:1, 8:1, 9:1, 10:1, 11:1, 12:1, 13:1, 14:1, 15:1, 16:1, 17:1, 18:1 or 19:1, etc.; however, the molar ratio is not limited to the listed values, and other unlisted values in the numerical range are also applicable.

As an optional technical solution in the present application, the first solvent in step (1) includes any one or a combination of at least two of methanol, ethanol, n-propanol, isopropanol, n-butanol, isobutanol, sec-butanol or tert-butanol. The typical but non-limiting combination examples include a combination of methanol and ethanol, a combination of ethanol and n-propanol, a combination of n-propanol and isopropanol, a combination of isopropanol and n-butanol, a combination of n-butanol and isobutanol, a combination of isobutanol and sec-butanol, a combination of sec-butanol and tert-butanol, a combination of tert-butanol and methanol or a combination of methanol, ethanol and isopropanol, etc.

Optionally, a molar ratio of the first solvent in step (1) to the 1,5-pentanediamine is (4-100):1, such as 5:1, 20:1, 30:1, 40:1, 50:1, 60:1, 70:1, 80:1 or 90:1, etc.; however, the molar ratio is not limited to the listed values, and other unlisted values in the numerical range are also applicable.

As an optional technical solution in the present application, the catalyst in step (1) includes any one or a combination of at least two of magnesium oxide, magnesium chloride, zinc oxide, zinc chloride, zinc acetate, zinc oxalate, zinc benzoate, vanadium pentoxide, vanadium trichloride, manganese dioxide, manganese tetrachloride, manganese acetate, manganese oxalate, iron (III) oxide, iron (III) chloride, iron acetate, iron oxalate, cobalt (III) oxide, cobalt trichloride, cobalt acetate, cobalt oxalate, nickel oxide, nickel acetate, nickel oxalate, copper oxide, copper chloride, molybdenum trioxide, molybdenyl acetylacetonate, tungsten trioxide, tungsten hexachloride, cerium oxide, cerium trichloride, cerium tetrachloride, lanthanum oxide, lanthanum trichloride, titanium dioxide, titanium tetrachloride or tetrabutyl titanate. The typical but non-limiting combination examples include a combination of magnesium oxide and magnesium chloride, a combination of zinc oxide and zinc chloride, a combination of zinc acetate and zinc oxalate, a combination of zinc oxalate and zinc benzoate, a combination of vanadium pentoxide and vanadium trichloride, a combination of manganese dioxide and manganese tetrachloride, a combination of manganese acetate and manganese oxalate, a combination of iron (III) oxide and iron (III) chloride, a combination of iron acetate and iron oxalate, a combination of cobalt (III) oxide and cobalt trichloride, a combination of cobalt acetate and cobalt oxalate, a combination of nickel oxide and nickel acetate, a combination of nickel acetate and nickel oxalate, a combination of copper oxide and copper chloride, a combination of molybdenum trioxide and molybdenyl acetylacetonate, a combination of tungsten trioxide and tungsten hexachloride, a combination of cerium oxide and cerium trichloride, a combination of cerium trichloride and cerium tetrachloride, a combination of lanthanum oxide and lanthanum trichloride, a combination of titanium dioxide and titanium tetrachloride or a combination of titanium tetrachloride and tetrabutyl titanate, etc.

Optionally, a mass of the catalyst in step (1) is 0.01-50% of a mass of the 1,5-pentanediamine, such as 0.01%, 0.05%, 0.1%, 0.2%, 0.5%, 1%, 2%, 5%, 10%, 15%, 20%, 25%, 30%, 35%, 40% or 45%, etc.; however, the mass is not limited to the listed values, and other unlisted values in the numerical range are also applicable.

As an optional technical solution in the present application, the carbonylation reaction in step (1) is carried out at 100-240 °C, such as 110 °C, 120 °C, 130 °C, 140 °C, 150 °C, 160 °C, 170 °C, 180 °C, 190 °C, 200 °C, 210 °C, 220 °C or 230 °C, etc; however, the temperature is not limited to the listed values, and other unlisted values in the numerical range are also applicable.

Optionally, the carbonylation reaction in step (1) is carried out at 1-24 h, such as 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h or 23 h, etc; however, the time is not limited to the listed values, and other unlisted values in the numerical range are also applicable.

In the present application, the solid-liquid separation can be carried out in a manner of filtration, centrifugation or sedimentation, etc., optionally filtration.

As an optional technical solution in the present application, the purification treatment in step (2) includes subjecting the pentane-1,5-dicarbamate reaction solution to ammonia removal, solvent removal and carbonylation agent removal.

Optionally, a solvent and a carbonylation agent obtained from the purification treatment are subjected back to step (1) for the carbonylation reaction.

In the present application, the ammonia removal is carried out in an ammonia removal tower, the solvent removal is carried out in a solvent removal tower, and the carbonylation agent removal is carried out in a carbonylation agent removal tower.

As an optional technical solution in the present application, the second solvent in step (2) includes any one or a combination of at least two of p-xylene, m-xylene, o-xylene, ethylbenzene, isopropylbenzene, butylbenzene, chlorobenzene or o-dichlorobenzene. The typical but non-limiting combination examples include a combination of p-xylene and m-xylene, a combination of m-xylene and o-xylene, a combination of o-xylene and ethylbenzene, a combination of ethylbenzene and isopropylbenzene, a combination of isopropylbenzene and butylbenzene, a combination of butylbenzene and chlorobenzene, a combination of chlorobenzene and o-dichlorobenzene, a combination of o-dichlorobenzene and p-xylene, or a combination of o-xylene, isopropylbenzene and o-dichlorobenzene, etc.

Optionally, the pentane-1,5-dicarbamate is 0.1-100% of a mass of the second solvent, such as 0.2%, 0.5%, 1%, 2%, 5%, 10%, 20%, 30%, 40%, 50%, 60%, 70%, 80% or 90%, etc.; however, the amount of the pentane-1,5-dicarbamate is not limited to the listed values, other unlisted values in the numerical range are also applicable.

As an optional technical solution in the present application, the thermal decomposition in step (2) is carried out in the presence of a catalyst.

In the present application, the thermal decomposition in step (2) can be carried out directly, and the addition of a catalyst can accelerate the reaction rate, shorten the reaction time, and reduce the formation of by-products.

Optionally, the catalyst includes any one or a combination of at least two of iron, nickel, cobalt, copper, molybdenum, titanium, antimony, tin or manganese, the typical but non-limiting combination examples include a combination of iron and nickel, a combination of nickel and cobalt, a combination of cobalt and copper, a combination of copper and molybdenum, a combination of molybdenum and titanium, a combination of titanium and antimony, a combination of antimony and tin, a combination of tin and manganese, a combination of manganese and iron, or a combination of nickel, cobalt and manganese, etc.

Optionally, an addition amount of the catalyst is 0.01-30% of a mass of the pentane-1,5-dicarbamate, such as 0.02%, 0.05%, 0.1%, 0.5%, 1%, 2%, 5%, 10%, 15%, 20% or 25%, etc.; however, the addition amount of the catalyst is not limited to the listed values, and other unlisted values in the numerical range are also applicable.

As an optional technical solution in the present application, the thermal decomposition in step (2) is carried out at 150-320 °C, such as 160 °C, 170 °C, 180 °C, 190 °C, 200 °C, 210 °C, 220 °C, 230 °C, 240 °C, 250 °C, 260 °C, 270 °C, 280 °C, 290 °C, 300 °C or 310 °C, etc.; however, the temperature is not limited to the listed values, and other unlisted values in the numerical range are also applicable.

Optionally, the thermal decomposition in step (2) is carried out at 0-5.0 MPa, such as 0 MPa, 0.02 MPa, 0.05 MPa, 0.1 MPa, 0.2 MPa, 0.5 MPa, 1.0 MPa, 1.5 MPa, 2.0 MPa, 2.5 MPa, 3.0 MPa, 3.5 MPa, 4.0 MPa or 4.5 MPa, etc.; however, the pressure is not limited to the listed values, and other unlisted values in the numerical range are also applicable.

Optionally, the thermal decomposition in step (2) is carried out for 0.5-24 h, such as 1 h, 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h or 23 h, etc.; however, the time is not limited to the listed values, and other unlisted values in the numerical range are also applicable.

As an optional technical solution in the present application, the method for preparing 1,5-pentane diisocyanate includes the following steps:
(1) mixing 1,5-pentanediamine, a carbonylation agent, a first solvent and a catalyst, performing a carbonylation reaction at 100-240 °C for 1-24 h, and performing solid-liquid separation to obtain a pentane-1,5-dicarbamate reaction solution;
   a molar ratio of the carbonylation agent to the 1,5-pentanediamine is (2-20): 1, a molar ratio of the first solvent to the 1,5-pentanediamine is (4-100):1, and a mass of the catalyst is 0.01-50% of a mass of the 1,5-pentanediamine; and
(2) subjecting the pentane-1,5-dicarbamate reaction solution obtained in step (1) to a purification treatment, and mixing pentane-1,5-dicarbamate after the purification treatment with a second solvent and a catalyst, and performing a thermal decomposition at 150-320 °C and 0-5.0 MPa for 0.5-24 h to obtain the 1,5-pentane diisocyanate;

the purification treatment includes subjecting the pentane-1,5-dicarbamate reaction solution to ammonia removal, solvent removal and carbonylation agent removal;
the pentane-1,5-dicarbamate is 0.1-100% of a mass of the second solvent, and an addition amount of the catalyst is 0.01-30% of a mass of the pentane-1,5-dicarbamate.

Compared with the prior art, the present application at least has the beneficial effects below.

The present application provides a method for preparing 1,5-pentane diisocyanate, the preparation method adopts a non-phosgene route, and has mild reaction condition, simple operation, no pollution and little potential safety hazard; the yield of PDC prepared in the present application can reach 99%, the purity is more than or equal to 99.5%, and the yield of PDI can be more than or equal to 95.0%. There is a good industrial application prospect.

### BRIEF DESCRIPTION OF DRAWINGS

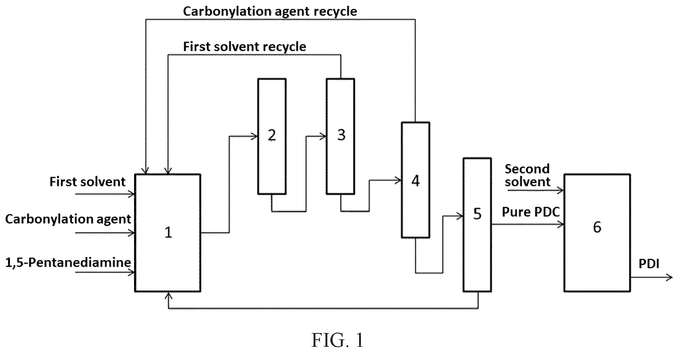

FIG. 1 is a system structure diagram showing the preparation for 1,5-pentane diisocyanate provided by embodiments in the present application;

Reference list: 1-carbonylation reactor, 2-ammonia removal tower, 3-solvent removal tower, 4-carbonylation agent removal tower, 5-PDC purification tower, 6-thermal decomposition reactor.

The present application will be further described in detail hereinafter. However, the following embodiments are only simple examples of the present application, and do not represent or limit the protection scope of the claims of the present application, the protection scope of the present application is defined by the appended claims.

### DETAILED DESCRIPTION

The technical solutions of the present application are further described below through specific embodiments.

For both a better explanation of the present application and a better understanding of the technical solutions of the present application, the typical but non-limiting embodiments of the present application are described below.

### Example 1

A method for preparing the 1,5-pentane diisocyanate of this example includes the following steps:
(1) 1,5-pentanediamine, ethyl carbamate, ethanol and an iron oxide catalyst were mixed and subjected to a carbonylation reaction at 190 °C for 5 h, the obtained system was filtered, and a pentane-1,5-dicarbamate reaction solution was obtained;
   a molar ratio of the ethyl carbamate to the 1,5-pentanediamine was 2:1, a molar ratio of the ethanol to the 1,5-pentanediamine was 20:1, and a mass of the iron oxide catalyst was 10% of a mass of the 1,5-pentanediamine; and
(2) the pentane-1,5-dicarbamate reaction solution obtained in step (1) was subjected to a purification treatment, pentane-1,5-dicarbamate after the purification treatment was mixed with chlorobenzene and a copper powder catalyst and subjected to a thermal decomposition at 250 °C and 1.3 MPa for 4 h, and the 1,5-pentane diisocyanate was obtained;

the purification treatment included subjecting the pentane-1,5-dicarbamate reaction solution to ammonia removal, solvent removal and carbonylation agent removal;
the pentane-1,5-dicarbamate was 5% of a mass of the chlorobenzene, and an addition amount of the copper powder catalyst was 10% of a mass of the pentane-1,5-dicarbamate.

The result showed that the conversion rate of PDA was 100%, the PDC yield was 99.0%, the product purity of PDC was 99.6%, and the PDI yield was 97.0%.

### Example 2

A method for preparing the 1,5-pentane diisocyanate of this example includes the following steps:
(1) 1,5-pentanediamine, n-butyl carbamate, n-butanol and a cerium trichloride catalyst were mixed and subjected to a carbonylation reaction at 200 °C for 5 h, the obtained system was filtered, and a pentane-1,5-dicarbamate reaction solution was obtained;
   a molar ratio of the n-butyl carbamate to the 1,5-pentanediamine was 15:1, a molar ratio of the n-butanol to the 1,5-pentanediamine was 20:1, and a mass of the cerium trichloride catalyst was 10% of a mass of the 1,5-pentanediamine; and
(2) the pentane-1,5-dicarbamate reaction solution obtained in step (1) was subjected to a purification treatment, pentane-1,5-dicarbamate after the purification treatment was mixed with p-xylene, a copper powder catalyst and a nickel powder catalyst for a thermal decomposition at 250 °C and 1.3 MPa for 4 h, and the 1,5-pentane diisocyanate was obtained;

the purification treatment included subjecting the pentane-1,5-dicarbamate reaction solution to ammonia removal, solvent removal and carbonylation agent removal;
the pentane-1,5-dicarbamate was 5% of a mass of the p-xylene, and an addition amount of the copper powder and an addition amount of the nickel powder were 5% of a mass of the pentane-1,5-dicarbamate, respectively.

The result showed that the conversion rate of PDA was 100%, the PDC yield was 99.4%, the product purity of PDC was 99.5%, and the PDI yield was 95.2%.

### Example 3

A method for preparing the 1,5-pentane diisocyanate of this example includes the following steps:
(1) 1,5-pentanediamine, diethyl carbonate, ethanol and a cerium trichloride catalyst were mixed and subjected to a carbonylation reaction at 120 °C for 5 h, the obtained system was filtered, and a pentane-1,5-dicarbamate reaction solution was obtained;
   a molar ratio of the diethyl carbonate to the 1,5-pentanediamine was 5:1, a molar ratio of the ethanol to the 1,5-pentanediamine was 20:1, and a mass of the cerium trichloride catalyst was 10% of a mass of the 1,5-pentanediamine; and
(2) the pentane-1,5-dicarbamate reaction solution obtained in step (1) was subjected to a purification treatment, pentane-1,5-dicarbamate after the purification treatment was mixed with o-dichlorobenzene and a copper powder catalyst for a thermal decomposition at 250 °C and 0.4 MPa for 4 h, and the 1,5-pentane diisocyanate was obtained;

the purification treatment included subjecting the pentane-1,5-dicarbamate reaction solution to ammonia removal, solvent removal and carbonylation agent removal;
the pentane-1,5-dicarbamate was 3.3% of a mass of the o-dichlorobenzene, and an addition amount of the copper powder was 10% of a mass of the pentane-1,5-dicarbamate.

The result showed that the conversion rate of PDA was 100%, the PDC yield was 98.0%, the product purity of PDC was 99.5%, and the PDI yield was 97.2%.

### Example 4

A method for preparing the 1,5-pentane diisocyanate of this example includes the following steps:
(1) 1,5-pentanediamine, urea, ethanol and lanthanum chloride catalyst were mixed and subjected to a carbonylation reaction at 190 °C for 24 h, the obtained system was filtered, and a pentane-1,5-dicarbamate reaction solution was obtained;
   a molar ratio of the dimethyl carbonate to the 1,5-pentanediamine was 2:1, a molar ratio of the ethanol to the 1,5-pentanediamine was 10:1, and a mass of the lanthanum chloride catalyst was 0.01% of a mass of the 1,5-pentanediamine; and
(2) the pentane-1,5-dicarbamate reaction solution obtained in step (1) was subjected to a purification treatment, pentane-1,5-dicarbamate after the purification treatment was mixed with isopropylbenzene and subjected to a thermal decomposition at 150 °C and 0.01 MPa for 24 h, and the 1,5-pentane diisocyanate was obtained;

the purification treatment included subjecting the pentane-1,5-dicarbamate reaction solution to ammonia removal, solvent removal and carbonylation agent removal;
the pentane-1,5-dicarbamate was 0.1% of a mass of the isopropylbenzene.

The result showed that the conversion rate of PDA was 100%, the PDC yield was 98.4%, the product purity of PDC was 99.5%, and the PDI yield was 95.0%.

### Example 5

A method for preparing the 1,5-pentane diisocyanate of this example includes the following steps:
(1) 1,5-pentanediamine, diisopropyl carbonate, isopropyl alcohol and vanadium trichloride were mixed and subjected to a carbonylation reaction at 240 °C for 1 h and then solid-liquid separation, and a pentane-1,5-dicarbamate reaction solution was obtained;
   a molar ratio of the diisopropyl carbonate to the 1,5-pentanediamine was 20:1, a molar ratio of the isopropyl alcohol to the 1,5-pentanediamine was 100:1, and a mass of the vanadium trichloride was 50% of a mass of the 1,5-pentanediamine; and
(2) the pentane-1,5-dicarbamate reaction solution obtained in step (1) was subjected to a purification treatment, pentane-1,5-dicarbamate after the purification treatment was mixed with ethylbenzene and tin powder and subjected to a thermal decomposition at 320 °C and 2.7 MPa for 0.5 h, and the 1,5-pentane diisocyanate was obtained;

the purification treatment included subjecting the pentane-1,5-dicarbamate reaction solution to ammonia removal, solvent removal and carbonylation agent removal;
the pentane-1,5-dicarbamate was 100% of a mass of the ethylbenzene; and an addition amount of the tin powder was 0.01% of a mass of the pentane-1,5-dicarbamate.

The result showed that the conversion rate of PDA was 100%, the PDC yield was 98.8%, the product purity of PDC was 99.5%, and the PDI yield was 95.2%.

It is seen from the results of the embodiments that the method for preparing 1,5-pentane diisocyanate by adopting non-phosgene route in the present application has the advantages of mild reaction condition, simple operation, no pollution, and little potential safety hazard. In the present application, the yield of PDC prepared can reach 99%, the purity is more than or equal to 99.5%, and the yield of PDI can be more than or equal to 95.0%. There is a good industrial application prospect.

The applicant declares that the present application illustrates the detailed structural characteristics of the present application through the above embodiments, but the present application is not limited to the detailed structural characteristics, which means that the present application is not necessarily rely on the detailed structural characteristics to be implemented.

Although the optional embodiments of the present application are described in detail herein, the present application is not limited to the specific details of the above embodiments. Within the scope of the technical conception of the present application, a variety of simple variations of the technical solutions in the present application can be made.

In addition, it should be noted that the specific technical features described in the above specific embodiments can be combined in any suitable ways as long as there is no contradiction. The practicable combinations will not be specified to avoid verboseness. Moreover, various embodiments in the present application can also be combined in any way.

## Claims

1. A method for preparing 1,5-pentane diisocyanate, comprising:
(1) mixing 1,5-pentanediamine, a carbonylation agent, a first solvent and a catalyst, performing a carbonylation reaction, and performing solid-liquid separation to obtain a pentane-1,5-dicarbamate reaction solution; and
(2) subjecting the pentane-1,5-dicarbamate reaction solution obtained in step (1) to a purification treatment, mixing pentane-1,5-dicarbamate after the purification treatment with a second solvent, and performing a thermal decomposition to obtain the 1,5-pentane diisocyanate.

2. The preparation method according to claim 1, wherein the carbonylation agent in step (1) has a structure as shown in Formula I: 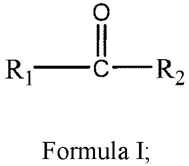 wherein Ri comprises any one of amino, methoxy, ethoxy, n-propoxy, isopropoxy, n-butoxy or isobutoxy, and R₂ comprises any one of amino, methoxy, ethoxy, n-propoxy, isopropoxy, n-butoxy, isobutoxy, sec-butoxy or tert-butoxy.

3. The preparation method according to claim 1 or 2, wherein a molar ratio of the carbonylation agent in step (1) to the 1,5-pentanediamine is (2-20): 1.

4. The preparation method according to any one of claims 1 to 3, wherein the first solvent in step (1) comprises any one or a combination of at least two of methanol, ethanol, n-propanol, isopropanol, n-butanol, isobutanol, sec-butanol or tert-butanol;
optionally, a molar ratio of the first solvent in step (1) to the 1,5-pentanediamine is (4-100): 1.

5. The preparation method according to any one of claims 1 to 4, wherein the catalyst in step (1) comprises any one or a combination of at least two of magnesium oxide, magnesium chloride, zinc oxide, zinc chloride, zinc acetate, zinc oxalate, zinc benzoate, vanadium pentoxide, vanadium trichloride, manganese dioxide, manganese tetrachloride, manganese acetate, manganese oxalate, iron (III) oxide, iron (III) chloride, iron acetate, iron oxalate, cobalt (III) oxide, cobalt trichloride, cobalt acetate, cobalt oxalate, nickel oxide, nickel acetate, nickel oxalate, copper oxide, copper chloride, molybdenum trioxide, molybdenyl acetylacetonate, tungsten trioxide, tungsten hexachloride, cerium oxide, cerium trichloride, cerium tetrachloride, lanthanum oxide, lanthanum trichloride, titanium dioxide, titanium tetrachloride or tetrabutyl titanate;
optionally, a mass of the catalyst in step (1) is 0.01-50% of a mass of the 1,5-pentanediamine.

6. The preparation method according to any one of claims 1 to 5, wherein the carbonylation reaction in step (1) is carried out at 100-240 °C;
optionally, the carbonylation reaction in step (1) is carried out for 1-24 h.

7. The preparation method according to any one of claims 1 to 6, wherein the purification treatment in step (2) comprises subjecting the pentane-1,5-dicarbamate reaction solution to ammonia removal, solvent removal and carbonylation agent removal;
optionally, the preparation method further comprises subjecting a solvent and a carbonylation agent obtained from the purification treatment back to step (1) for the carbonylation reaction.

8. The preparation method according to any one of claims 1 to 7, wherein the second solvent in step (2) comprises any one or a combination of at least two of p-xylene, m-xylene, o-xylene, ethylbenzene, isopropylbenzene, butylbenzene, chlorobenzene or o-dichlorobenzene;
optionally, the pentane-1,5-dicarbamate is 0.1-100% of a mass of the second solvent.

9. The preparation method according to any one of claims 1 to 8, wherein the thermal decomposition in step (2) is carried out in the presence of a catalyst;
optionally, the catalyst in step (2) comprises any one or a combination of at least two of iron, nickel, cobalt, copper, molybdenum, titanium, antimony, tin or manganese;
optionally, an addition amount of the catalyst in step (2) is 0.01-30% of a mass of the pentane-1,5-dicarbamate.

10. The preparation method according to any one of claims 1 to 9, wherein the thermal decomposition in step (2) is carried out at 150-320 °C;
optionally, the thermal decomposition in step (2) is carried out at 0-5.0 MPa;
optionally, the thermal decomposition in step (2) is carried out for 0.5-24 h.

11. The preparation method according to any one of claims 1 to 10, comprising:
(1) mixing 1,5-pentanediamine, a carbonylation agent, a first solvent and a catalyst, performing a carbonylation reaction at 100-240 °C for 1-24 h, and performing solid-liquid separation to obtain a pentane-1,5-dicarbamate reaction solution;
a molar ratio of the carbonylation agent to the 1,5-pentanediamine is (2-20): 1, a molar ratio of the first solvent to the 1,5-pentanediamine is (4-100):1, and a mass of the catalyst is 0.01-50% of a mass of the 1,5-pentanediamine; and
(2) subjecting the pentane-1,5-dicarbamate reaction solution obtained in step (1) to a purification treatment, mixing pentane-1,5-dicarbamate after the purification treatment with a second solvent and a catalyst, and performing a thermal decomposition at 150-320 °C and 0-5.0 MPa for 0.5-24 h to obtain the 1,5-pentane diisocyanate;
the purification treatment comprises subjecting the pentane-1,5-dicarbamate reaction solution to ammonia removal, solvent removal and carbonylation agent removal;
the pentane-1,5-dicarbamate is 0.1-100% of a mass of the second solvent, and an addition amount of the catalyst is 0.01-30% of a mass of the pentane-1,5-dicarbamate.
